# EUROPEAN PATENT APPLICATION

(11) **EP 1 891 938 A1**
(43) Date of publication of application: **27.02.2008**
(21) Application number: 06291198.7
(22) Date of filing: 24.07.2006
(51) Int. Cl.: A61K 9/00, A61K 9/16

(54) **High dose orally dissolvable/disintegrable lyophilized dosage form**

(71) Applicant: CEPHALON FRANCE, 94704 Maisons-Alfort Cedex (FR); Cima Labs, Inc., Eden Prairie, Minnesota 55344-9361 (US)
(72) Inventor: Khankari, Rajendra, Maple Grove, MN 55331 (US); Moe, Derek, Maple Grove, MN 55311 (US); Hamed, Ehab, Maple Grove, MN 55311 (US); Nguyen, Tam, 94450 Limeil-Brevannes (FR)
(74) Representative: Colombet, Alain André

(57) **Abstract**

The present invention provides an orally dissolvable/disintegrable, lyophilized dosage form including a protected granulate comprising an active ingredient and a protective granulation binder, which substantially protects the form and/or attributes of a granulate and/or active ingredient, and participates in masking bad tasting active ingredients, such as sulfur-containing materials, and a matrix, and a method for making same. There is also provided a method of treating a patient using any orally dissolvable/disintegrable lyophilized dosage form comprising the steps of placing the dosage form in the mouth of a patient in need of treatment, allowing the dosage form to disintegrate/dissolve sufficiently to allow it, and in particular, the protected granulate, to be swallowed as a solution, suspension or slurry, and swallowing the at least partially disintegrated/dissolved dosage form.

## Description

### BACKGROUND OF THE INVENTION

Conventional tablets manufactured using common tablet compression and coating techniques entail the use of relatively large percentages of excipients in addition to the active agent(s). Such excipients may include fillers, binders, lubricants, anti-adherents, glidants, colorants, polymer coatings, plasticizers, disintegrants, etc. The excipient levels can, in some cases, exceed 90% of the tablet weight, depending on the drug properties and the intended use of the tablet. Accordingly, for bitter tasting drugs with dosage levels above 50mg, tablet weight can be higher than 1.5g, rendering them questionable for human consumption, especially for geriatric and pediatric patients. The use of lyophilization as an alternative manufacture technique offers the advantage of requiring the use of less and fewer excipients.

Lyophilized dosage forms are known. See for example Lafon, U.S. Patent No. 4,616,047, Nguyen et al., U.S. Patent No. 5,843,347 and Blonde et al., U.S. Patent No. 3,855,712. These lyophilized dosage forms can be based on lyophilized emulsions such as that described in the '047 patent, and these formulations may include natural or synthetic sweetening agents such as saccharose, glucose, xylose, sorbitol, saccharine and saccharinates (especially the sodium, potassium and calcium saccharinates), the cyclamates (especially the sodium, potassium and calcium cyclamates), aspartame and taste modifying agents (in particular for the purpose of concealing the bitter after-taste of synthetic sweeteners of the saccharine and cyclamate type) such as in particular the citric, ascorbic and tartaric acids and aromas. The '347 patent describes, amongst other things, lyophilized microbeads which may then be coated with a host of compounds including polyacrylics and polymethacrylic acid esters ethyl/vinyl acetate copolymers, polymethylcyloxanes, polyacrylamides, PVP and polyvinyl acetate, polylactic and polyglycolic acids and copolymers thereof, polyurethanes, polypeptides and others. In one embodiment, the coating produces a microporous semipermeable membrane allowing the contained material to dissolve wherein an osmotic pressure is created which expels the aqueous solution containing the active ingredient. These formulations can include various diluents, sweeteners and artificial sweeteners, as well as natural or synthetic flavorings and mixtures thereof. See also U.S. Patent Nos. 4,490,407 and 3,939,260.

In non-lyophilized formulations, it is known to use various coating materials for taste masking. See for example U.S. Patent Nos. 5,178,878, 5,223,264, 6,024,981, and 6,740,341, all of which are assigned to CIMA LABS INC. of Eden Prairie, Minnesota.

However, a problem exists when using taste masking coating technology in the preparation of lyophilized dosage forms. Often the ingredients contained in a lyophilized dosage form are suspended, dispersed or even dissolved in lyophilization solvents which can include, for example, water, short chain normal alcohols, and the like. The lyophilization process can extend over a number of hours so solvent exposure can be prolonged. Sometimes these ingredients can be adversely affected by such solvent exposure, particularly prolonged exposure. Indeed, materials could unintentionally dissolve, thereby destroying some desirable attribute. A dissolved granulate, for example, is no longer a granulate. Similarly, with taste masked dosage forms, the taste masking coating could be compromised and thus the taste masking as well. Thus, there still remains a significant need for improvements in technology of producing dosage forms.

Moreover, it is known that some active ingredients found in pharmaceuticals, dietary supplements, etc. have a taste that is hard to mask in chewable, dissolvable and disintegrable dosage forms. The taste-masking problem is compounded by the fact that many of these ingredients must be administered in high dosages in order to treat a patient with a therapeutically effective amount. In order to mask bad tasting active ingredients, such as sulfur-containing products, manufacturers have resorted to applying various coating materials as noted above. However, this increases the size of the granule, and thereby decreases the amount of active that can be incorporated in any dosage form.

### SUMMARY OF THE INVENTION

The present invention provides, in one embodiment, an orally dissolvable/disintegrable, lyophilized dosage form including a protected granulate comprising an active ingredient and a protective granulation binder, which substantially protects the form and/or attributes of a granulate and/or active ingredient, and participates in masking bad tasting active ingredients, such as sulfur-containing materials. The dosage form also includes a matrix. The term "protected granulate" means a granulate incorporating at least one protective granulation binder and/or active ingredient. A "protective granulation binder" is a granulation binder which can preserve the granulate and/or the active ingredient, in substantially the desired form or structure that the granulate and/or active ingredient had prior to being contacted with a lyophilization solvent. This includes without limitation, preserving particle size, crystalline or amorphous form, structure, state of granulation, color or fragrance, or the like. Specifically, in a preferred embodiment, no more than 10% of the active ingredient will dissolve during the lyophilization process and most preferably no more than 5%. This can be measured by centrifuging the suspension at certain times and measuring the solution concentration of the API. The balance of the dosage form is an extra-granular matrix also referred to herein simply as the "matrix". In preferred embodiments, this invention may be used to produce dosage forms containing a relatively high dose of an active ingredient. The dosage forms of the present invention are intended for direct oral dosing which means that they will be placed into the mouth as a solid to be dissolved/disintegrated in the mouth and thereafter the resulting solution, dispersion, suspension or slurry is swallowed.

In one embodiment, the dosage form comprises a granulate with at least one active ingredient and at least one protective granulation binder. This is also referred to herein as a "protected granulate." The protected granulate may also include coating materials, solid supports, additional taste masking substances, additional granulation binders, pH adjusting substances or buffers, and other excipients as described herein.

The matrix may comprise one or more of a binder, lyophilization binder, filler, sugar, artificial sweetener, polymer, flavoring agent, taste masking material, active ingredient, coloring agent, lubricant, effervescent disintegrant, non-effervescent disintegrant, viscosity modifier, surfactant and buffer. This matrix is usually lyophilized as well, often along with the protected granulate.

In another embodiment, the dosage form comprises at least one sulfur containing active ingredient.

In yet another embodiment, the dosage form contains at least one flavor selected to be complimentary with the active ingredient so as to provide a pleasant organoleptic sensation in the mouth. Some flavors include, but are not limited to fruit punch, orange, banana, cherry, mint, bubble gum, grape, paradise punch, peach, mango, and combinations thereof. Particularly useful for sulfur containing, or bad-tasting drugs and, in particular Modafinil, famotidine or fentanyl and/or salts thereof are flavors including mint and fruit punch.

Processes of making the dosage forms of the invention in some instances allow for the inclusion of high doses of active ingredients and forms of active ingredients which were not previously possible as well as the realization of lyophilized dosage forms never previously thought possible. These processes are also considered to be a part of the invention.

The present invention also allows for at least one of rapid delivery of an active ingredient into the digestive process, or reduction in the residence time of that active ingredient on the tongue compared to some other in-mouth disintegrable tablets, and still provides a pleasant organoleptic sensation in the mouth.

In yet another embodiment of the present invention, there is provided a method of treating a patient using any orally dissolvable/disintegrable lyophilized dosage form described herein comprising the steps of placing the dosage form in the mouth of a patient in need of treatment, allowing the dosage form to disintegrate/dissolve sufficiently to allow it, and in particular, the protected granulate, to be swallowed as a solution, suspension or slurry, and allowing the patient to swallow the at least partially disintegrated/dissolved dosage form. This method is particularly useful for persons who cannot or will not swallow, geriatric patients, mental patients, and children.

### DETAILED DESCRIPTION

The "dosage form(s) adapted for direct oral dosing" or more simply, the "dosage form(s)" of the invention are defined as solids such as tablets, capsule, caplets, wafers, films and the like adapted to rapidly dissolve/disintegrate in the mouth. Most preferred are tablets, caplets and wafers. After the dosage form dissolves and/or disintegrates in the mouth, the resulting solution, slurry or suspension is swallowed such that the active ingredient can enter the digestive tract down stream of the mouth. The active ingredient then enters the blood stream via, for example, the stomach and/or intestines.

"Orally dissolve(able)/disintegrate(able)" means that the water soluble ingredients within the dosage form will dissolve and/or disintegrate sufficiently to allow ingestion as a solution, suspension or slurry. Most preferably, the solution, suspension or slurry will be considered generally non-gritty, and will be pleasant in terms of viscosity and mouth feel by taste tests according to those of skill in the art.

It should be understood that whether a dosage form in accordance with the invention can be considered "dissolvable" or "disintegrable" or both depends upon, at least, the nature of the dosage form, the load of active ingredient, the size of the dosage form and the materials used. "Dissolving" in accordance with the present invention is a process similar to melting in one's mouth and depends upon a number of factors, most importantly, the degree of water solubility of the ingredients in question and how much of the dosage form is composed of highly water-soluble ingredients. This involves the nature of the material (its inherent solubility in water at 25 degrees C. (in a glass) or 37 degrees C. (in the mouth)) as well as things like particle size, average particle size, porosity, form (crystalline, amorphous, solid solution) and the like. However, it will be appreciated that a material that is inherently not water soluble will not suddenly become water soluble merely by, for example, reducing its average particle size. "Disintegration" and like terms mean that the dosage form falls apart into smaller particles and/or aggregates. As will be appreciated, when a dosage form in accordance with the present invention is placed in the mouth, and preferably on the tongue, portions of the dosage form will break down and others will begin to dissolve in the period between being placed in the mouth and being swallowed. Indeed, some materials will both break apart and begin to dissolve. Unless the context suggests otherwise the use of either "dissolve" or "disintegrate" is meant to imply the possibility of either or both processes.

The dosage forms of the invention preferably will rapidly dissolve and/or disintegrate sufficiently in the mouth such as, for example, within 120 seconds or less, preferably 60 seconds or less, more preferably 40 seconds or less, and even more preferably 30 seconds or less, so as to allow them to be swallowed as a solution, suspension or a slurry containing the protected particles. In some embodiments, disintegration/dissolution occurs in about 10 seconds or less. Preferably, the dosage forms also provide a pleasant organoleptic sensation. Generally, if the dosage form will disintegrate in a USP disintegration apparatus within 60 seconds or less, it is likely to meet the in-mouth dissolution/disintegration standards described herein. See 29/24 USP/NF 2671. This test is used to determine whether dosage forms disintegrate within a prescribed time when placed in a liquid medium under certain conditions. Preferably, the dosage forms described herein also provide a pleasant organoleptic sensation.

"Pleasant organoleptic sensation" is used here to mean that the oral dosage form does not provide a relatively gritty sensation, has a good mouth feel in terms of viscosity, cohesion of the particulate and the like and provides adequate taste masking so as to make ingestion palatable and preferably pleasing. As contemplated by the present invention, the dosage form will be small enough, will encourage saliva secretion and/or will dissolve/disintegrate in a short amount of time, reducing the residence time in the mouth any or all of which aid in providing a pleasant experience.

"Solution, slurry, or suspension" means that the dosage form is mixed with an adequate amount of liquid (water/saliva) so that the dosage form dissolves/disintegrates adequately to create a solution or a liquid containing suspended solids, which can easily be swallowed.

"Protective granulation binder" in accordance with the present invention means an ingredient incorporated with an active ingredient into a granulate which provides sufficient protection thereto from lyophilization solvents, and also provides taste masking capabilities. In particular, it is meant to stop the unintended and undesired leakage of the active ingredient from the dosage form and/or the granulate. In a most preferred aspect, but one which is not necessary for the functioning of the invention, the protective granulation binder should substantially preserve at least one of the structural integrity, function and/or physical attributes of the active ingredient, the granulate during the lyophilization process and thereafter. These protective granulation binders themselves also provide taste masking and may impart additional advantages. Most importantly, however, the protective granulation binder will provide sufficient protection for the active ingredient and/or granulate, as described above, during lyophilization but will impart little or no "unintended change" to the intended release of the active ingredient once a dosage form (or protected granulate) containing same clears a patient's mouth.

There are generally two tests that are used to determine "leakage" of a dosage form. In the first, a microscope is used to evaluate the dosage form or granulate. The dosage form, or, as appropriate, the granulate, is placed into a solution for a length of time, generally 2 to 4 hours, then removed from the solution and inspected under a microscope. While this test will show swelling, and general changes to the exterior of the dosage form, it is not an accurate detection of leakage and is therefore not the recommended test. The second, quantitative test requires placing the dosage form, or, as appropriate, the granulate, into a solution for a length of time, again generally 2 to 4 hours, centrifuging the sample, removing the supernatant and quantitatively measuring for the amount of active ingredient which may have been released. The second test is more accurate in determining any change to the dosage form, including any release of the active ingredient. Thus, for the present invention, the second, quantitative test is the method of determining leakage.

In a preferred embodiment of the present invention, the amount of leakage, as measured by the quantitative test, is preferably less than 20%, and more preferably less than 10%, and most preferably less than 5%, depending on the solubility of the active ingredient and the amount of protective granulation binder. In accordance with the present invention, when the active ingredient is solvent insoluble, it is preferred that the leakage is less than 10% and more preferably less than 5%. It is also contemplated by the present invention that when a solvent soluble active ingredient is used, an increase in the amount of protective granulation binder, and/or control of pH and/or ionic strength of the solvent may also be manipulated to reduce leakage.

In a preferred embodiment, the protective granulation binder is selected such that it will not dissolve in the lyophilization solvent, or at least dissolves sufficiently slowly to provide substantial protection during exposure to the lyophilization solvent. It is also preferred that the active ingredient be selected such that it will be substantially protected by the protective granulation binder. More preferably, the protective granulation binder is selected to be used in conjunction with an active that is substantially insoluble in the selected lyophilization solvent.

"Solvent insoluble" in accordance with the present invention means that insoluble ingredients within the protected granulate and/or the dosage form will not readily dissolve or disintegrate sufficiently when contacted with a lyophilization solvent for a period of between 1 and 6 hours, and more preferably a period of between 2 and 4 hours. Again, this can be measured by use of a centrifuge followed by measuring solution concentration. While it is preferred that the protective granulation binder and active ingredient be selected based on its solvent insolubility, it will be appreciated that such selection depends upon a number of factors, including, but not limited to, the taste of the active ingredient, the amount of active ingredient and/or protective granulation binder, the ratio of active ingredient to protective granulation binder, the degree of taste masking imparted to the dosage form by the matrix, or other ingredients added to the protected granulation binder, or coatings added to the dosage form, etc. Thus, it is contemplated by this invention that the active ingredient(s), and/or other ingredients within the dosage form may be solvent insoluble, partially solvent insoluble, or solvent soluble. In addition, it is also contemplated by this invention that the lyophilization solvent may be selected to reduce solubility of the LSP coating, active ingredient, and/or the dosage form, including controlling pH levels and ionic strength of the lyophilization solvent to help prevent leakage or change.

"Effervescent" means that the dosage form, when mixed with liquid, including water and saliva, will evolve a gas. Preferred effervescent agents evolve gas by means of a chemical reaction which takes place upon exposure of the effervescent disintegration agent to water and/or to saliva in the mouth. This reaction is most often the result of the reaction of a soluble acid source and an alkali monocarbonate or carbonate source. The reaction of these two general compounds produces carbon dioxide gas upon contact with water or saliva. Such water-activated materials must be kept in a generally anhydrous state and with little or no absorbed moisture or in a stable hydrated form, since exposure to water will prematurely disintegrate the tablet. Of course, an effervescent couple (or the individual acid and base separately) can be coated with a coating to prevent premature reaction.

The dosage forms of the present invention includes a granulate comprising at least one active ingredient and at least one protective granulation binder, and a matrix. The granulate comprises at least one first active ingredient, although it can include other active ingredients or any other pharmaceutical excipients as well. These can include solid supports, taste masking agents, beads, binders, disintegrants, pH adjusting substances, buffers, and the like. The granulate is preferably present in one or more dosage forms in an amount sufficient to provide a therapeutically effective amount of said at least one active ingredient. Preferably, the dose is found in two or fewer dosage forms, and most preferably in one dosage form.

A "therapeutically effective amount" is the amount or quantity of a drug or active ingredient which is sufficient to elicit the required or desired therapeutic response, or in other words, the amount which is sufficient to elicit an appreciable biological response when administered to a patient. The does need not be optimal, nor even provide a cure or symptomatic relief. As used with reference to a vitamin or mineral, the term "effective amount" means an amount at least about 10% of the United States Recommended Daily Allowance ("RDA") of that particular ingredient for a patient. For example, if an intended ingredient is vitamin C, then an effective amount of vitamin C would include an amount of vitamin C sufficient to provide 10% or more of the RDA.

The granulate includes at least one first active ingredient. "Active ingredients" in accordance with the present invention include materials capable of being lyophilized, or otherwise useful in the present invention. Such active ingredients may include systematically distributable pharmaceutical ingredients, vitamins, minerals, dietary supplements, as well as nonsystemically distributable drugs. A combination or mixture of any of the foregoing is also contemplated by the present invention. Pharmaceutical ingredients may include, without limitation, antacids, analgesics, anti-inflammatory, antibiotics, diuretics, anorexics, antihistamines, antiasthmatics, antidiuretics, antiflatuents, antimigraine agents, antispaspodics, sedatives, antihyperactives, antihypertensives, tranquilizers, decongestants, immunosuppressants, anticancers, antivirals, antiparasitics, antifungals, antiemetics, antidepressants, antiepileptics, local anesthetics, vasoactive agents, antiasthmatics, skeletal muscle relaxants, drugs for parkinsonism, antipsychotics, hematopoietic growth factors, antihyperlipidemics, anticoagulants, fibrinolytics, antithrombotics, hormones, therapeutic proteins and peptides, antiarrhythmia, antiangina, beta blockers and combinations thereof. In one embodiment in accordance with the present invention, the active ingredients are preferably pharmaceutical agents that are substantially lyophilization solvent insoluble and having a bitter taste at high dosage, including sulfur containing compounds such as Modafinil or its salts. Fentanyl and its salts are also preferred as active ingredients.

As used in this disclosure, the term "vitamin" refers to trace organic substances that are required in the diet. For the purposes of the present invention, vitamin(s) include, without limitation, thiamin, riboflavin, nicotinic acid, pantothenic acid, pyrdoxine, biotin, folic acid, vitamin B12, lipoic acid, ascorbic acid, vitamin A, vitamin D, vitamin E and vitamin K. Most preferred are those vitamins that are insoluble in the selected lyophilization solvent. Also included within the term vitamin are the coenzymes thereof. Coenzymes are specific chemical forms of vitamins. Coenzymes that may be useful in the present invention include thiamine pyrophosphates (TPP), flavin mononucleotide (FMM), flavin adenine dinucleotive (FAD), Nicotinamide adenine dinucleotide (AND), Nicotinamide adenine dinucleotide phosphate (NADP) Coenzyme A (CoA) pyridoxal phosphate, biocytin, tetrahydrofolic acid, coenzyme B.sub.12, lipoyllysine, 11-cis-retinal, and 1,25-dihydroxycholecalciferol. The term vitamin(s) also includes choline, carnitine, and alpha, beta, and gamma carotenes.

As used in this disclosure, the term "mineral" refers to inorganic substances, metals, and the like required in the human diet. Thus, the term "mineral" as used herein includes, without limitation, calcium, iron, zinc, selenium, copper, iodine, magnesium, phosphorus, chromium and the like, and mixtures thereof.

The term "dietary supplement" as used herein means a substance which has an appreciable nutritional effect when administered in small amounts. Dietary supplements include, without limitation, such ingredients as bee pollen, bran, wheat germ, kelp, cod liver oil, ginseng, and fish oils, amino-acids, proteins and mixtures thereof. As will be appreciated, dietary supplements may incorporate vitamins and minerals.

While at least one active ingredient is desired, multiple active ingredients may also be used. So too the dosage form may include lyophilized and non-lyophilized active ingredients. One active may be coated and the other uncoated. Any amount of active ingredients as described herein are possible, so long as there is at least some amount of active ingredient in accordance with the present invention as part of a lyophilized granulate.

The amount of active ingredient used can vary greatly and can depend upon, among other things, the type and properties of the active ingredient, the condition it is intended to treat, the size and type of the patient, the size and nature of the dosage form, whether or not more than one active ingredient is to be delivered from the dosage form and how many dosage forms will be used to deliver each dose. Generally, the total amount of active ingredient for any individual dosage form is in a therapeutically effective amount, and in certain embodiments, the total amount of active ingredient is in an amount of about 0.01 mg to about 2.0g by weight, more preferably about 0.05mg to about 1g, even more preferably about 1 mg to about 800mg, and most preferably about 85mg to about 425mg. This is based on the amount of active ingredient only -- not counting, for example, the amount of protective granulation binder, and/or any other coating or excipient in the final dosage form. In terms of the proportion of the granulate that is active ingredient, the active ingredient can range from about 0.1% to about 99.9% by weight of the granulate, and more preferably in an amount of about 50% to about 98% by weight of the granulate, and most preferably in an amount of about 85% to about 95% by weight of the granulate. The balance would be any coating(s), protective granulation binders, and excipients.

The protective granulation binder that is used to substantially protect an active ingredient and/or granulate in accordance with the present invention from dissolution or degradation while in contact with lyophilizing solvents often makes up the balance of the weight percentage of the protected granulate. In other instances, the balance includes other coatings and/or excipients and the like.

The protective granulation binder also acts as a taste masking agent for bad tasting active ingredients. Taste masking can be measured by placing a dosage form in the mouth and retaining it in the mouth until it dissolves/disintegrates in the mouth to the point where it would be swallowed. It may be swallowed or spit-out at that point.

The protective granulation binder generally is made from any natural or synthetic polymer including: acrylic polymers, modified celluloses, and the like, which are pH dependant materials. In one embodiment, the binder materials will become soluble at a pH which is generally acidic (pH 7 or below) and in another embodiment the binder materials will become soluble at a pH which is generally basic (pH 7 or above). In a third embodiment, the materials become soluble at a generally neutral pH (pH 6-8). There are a number of factors which determine which material is used as a protective granulation binder in a given situation including, without limit, ease of handling and processing, cost, thickness, site of intended dissolution of the protective granulation binder and/or the active ingredient, and the type and pH of the lyophilization solvent to be used.

In one preferred embodiment, the protective granulation binder is made from a material that becomes soluble at a pH of about 6.5 or below. In another embodiment, the binder becomes soluble at a pH of between about 6.0 and about 6.5. These polymers and copolymers should preferably be pharmacologically acceptable, capable of providing appropriate release and while still being convenient to process. These include, for example, acrylic polymers, modified celluloses amino alkyl acrylate copolymers such as, for example, copolymers of methylmethacrylate, butylmethacrylate and dimethylaminoethyl methacrylate. *See European Pharmacopoeia* 4.4 (04/2003:1975) at 3385. In one particularly preferred embodiment, the copolymer has a relative molecular mass of about 150,000 and a ratio of dimethylaminoethyl methacrylate groups to butylmethacrylate groups and methylmethacrylate groups of about 2:1:1 and the content of the dimethylaminoethyl groups is about 20.8% to 25.5% based on the amount of dry substances present.

A particularly preferred material can be obtained under the mark Eudragit E-100, which can be used in normal form or in micronized Eudragit E-100 and mixtures thereof. Eudragit is a trademark of Rohm GmbH, Chemische Fabrik, Kirschenallee, D-64293, Darmstadt, Germany for a group of acrylic polymers.

These materials are generally solid at room temperature. However, they may be applied by being dissolved, suspended, emulsified, dispersed or the like in a solvent or solvent system. Preferred solvents in accordance with the present invention include those capable of substantially dissolving or dispersing Eudragit E-100 such as water, normal C₁-C₅ alcohol, branched C₁-C₅ alcohol, denatured C₁-C₅ alcohol, and low molecular weight ketones such as acetone and MEK. Ethanols, including (SDA-3A) and denatured ethanol are most preferred.

In the dosage forms of the invention, the protected granulate is generally present in the dosage form in an amount sufficient to provide a therapeutically effective amount of an active ingredient, with the balance of the dosage form being the matrix. Generally, the amount of protected granulate is preferable about 0.1 % to about 90%, and more preferably about 1% to about 80% by weight of the dosage form. Generally, the amount of active ingredient is preferably about 0.01mg to about 2g by weight of the dosage form, and more preferably about 0.05mg to about 1g, and even more preferably about 1mg to about 800mg, and most preferably about 85mg to about 425mg. One of the advantages of the invention is that, in some embodiments, the dosage form includes a relatively high dose of active ingredient (> 50 mg for bitter drugs), for example, one dosage form contemplated by the present invention may include between 85 mg and 425 mg of Modafinil. Yet the resulting dosage form remains a palatable size or can be divided into fewer dosage forms for a given dose.

Generally the total amount of protective granulation binder within the protected granulate for any individual dosage form is in an amount of about 0.1 % to about 50% by weight of the protected granulate, and more preferably in an amount of about 2% to about 25% by weight of the protected granulate, and most preferably in an amount of about 5% to about 15% by weight of the protected granulate. (In these instances, "granulate" includes the weight of the active ingredient and any other taste masking, controlled release or delayed release coatings and any excipients and is measured as percent weight gain based on a finite sample of granulate.) Of course, these percentages are dependent upon the kind(s) of active ingredient used, the kind(s) and type(s) of protective granulation binders used, the kind or type of taste masking agent(s) used, the concentration of the taste masking agent, the amount of active agent to be delivered in the dose, etc.

It is also contemplated that another ingredient may be added to the granulate before or during the granulation or milling process, including but not limited to fillers, taste-masking agents, disintegrates, binders, flavors, etc. Any other ingredient may be added in an amount of about 0% to 99.5% by weight of the granulate.

The dosage form of the invention also includes a matrix, making up the balance of the dosage form. The matrix comprises at least one of a binder, lyophilization binder, filler, sugar, artificial sweetener, polymer, flavoring agent, taste masking material, active ingredient, coloring agent, lubricant, effervescent disintegrant, non-effervescent disintegrant, viscosity modifier, surfactant and buffer. Any conventional material may be used to provide a matrix in accordance with the present invention, so long as it meets the overall objectives of the present invention, including dissolvability/disintegratability in the mouth. The amount of any one or more of these ingredients will vary with the amount of active ingredient, protected granulate size, shape of the dosage form, rate of dissolution/disintegration, the need for complimentary taste masking, how many ingredients are used, which ingredients are used, etc. Any combination or amounts are contemplated sufficient to allow the creation of a soluble, storable dosage form in accordance with the present invention, particularly one exhibiting a pleasant organoleptic sensation.

Binders can be anything known to be used as binders. Some binders that may be useful in the present invention include acacia, tragacanth, gelatin, starch, cellulosic materials such as methyl cellulose, sodium carboxymethylcellulose, hydroxypropylmethylcellulose, hydroxypropylcellulose, hydroxyethylcellulose, polyvinyl pyrrolidone, alginic acids and salts thereof, magnesium aluminum silicate, polyethylene glycol, guar gum, polysaccharide acids, bentonites, sugars, invert sugars, xanthan gum, Dextran 20, 40 70, povidone, copovidone, cyclodextrines and derivatives, and the like. Binders can be used in conventional amounts and preferably in an amount of about 0.2% by weight to about 20% and more preferably about 2% to about 10% percent by weight of the total dosage form.

Lyophilization binders can be anything known to be used as a lyophilization binder. Some lyophilization binders that may be useful in the present invention include Dextran 70, povidone, and methyl cellulose. Lyophilization binders can be used in conventional amounts and preferably in an amount of about 2% to about 10%, and more preferably from about 4% to about 8%.

Fillers can be anything known to be used as fillers. Some fillers that may be useful in the present invention include mannitol, dextrose, sorbitol, lactose, sucrose, and calcium carbonate. Fillers can be used in conventional amounts and preferably in an amount of about 0.5% to about 99%, and more preferably about 5% to about 50%.

A particularly preferred type of filler which may be used is sugars. Sugars that may be used in the present invention include sugar, sugar alcohols, ketoses, saccharides, polysaccharides, oligosaccharides and the like, as well as celluloses and modified celluloses.

Sugars may also include direct compression and/or nondirect compression sugars. Particularly preferred nondirect compression sugars include, without limitation, dextrose, mannitol, sorbitol, trehalose, lactose and sucrose. Of course, these sugars generally exist as either a direct compression sugar, *i.e.,* a sugar which has been modified to increase its compressibility and/or flow, or a nondirect compression sugar which does not have sufficient flowability and/or compressibility to allow it to be used in high speed processing and multi-tablet presses without some sort of augmentation such as, without limitation, a glidant to increase flow, granulation to increase flow and/or compressibility and the like. Of course, techniques like granulation can also be used to convert something which initially has sufficient flow and compressibility to be considered a direct compression sugar before processing into a nondirect compression sugar as well. This can be measured by directly compressing tablets made only from a sugar and comparing the flow and compressibility both before and after processing. If flow and/or compressibility are reduced after processing the material is likely to have become a nondirect compression sugar. It will be appreciated however, that whether or not the reduction in properties are sufficient to require augmentation or further processing before the sugar is used in a commercial process will depend on a number of factors including the amount used, the type of processing equipment used, and the overall formulation. Generally, however, some further processing or augmentation is required. While not definitive, sometimes a nondirect compression sugar will have at least about 90% of its particles smaller than about 200 microns, and more preferably 80% smaller than about 150 microns.

The amount of total sugar can range from about 0.5% to about 95%. More preferably, the amount of sugar will range from about 5% to about 50%, and even more preferably between about 5% and 25%.

Artificial sweeteners can be anything known to be used as artificial sweeteners. Some artificial sweeteners that may be useful in the present invention without limitation include saccharin, aspartame, sucralose, neotame, and acesulfame potassium. Artificial sweeteners may be used in conventional amounts, and preferably in an amount ranging from about 0.1 % to about 10%.

Polymers may include natural or synthetic polymer including acrylic polymers, modified celluloses, and the like. In one embodiment, the coating materials are pH dependant materials that become soluble at a pH of 6.0-6.5 or below such as EUDRAGIT, from Rohn & GmbH, Chemische Fabrik, Kirschenallee, D-64293 Darmstadt, Germany, a group of acrylic polymers including for example, without limitation EUDRAGIT E100, modified celluloses such as ethyl cellulose, methyl cellulose, hydroxypropylcellulose, sodium carboxy methyl cellulose, hydroxyethylcellulose, polyoxymer, and/or hydroxypropylmethylcellulose. Polymers may be used in conventional amounts, and preferably in an amount ranging from about 0% up to about 15%.

Flavoring agents can be anything known to be used as flavoring agents. Flavoring agents that may be useful in the present invention may include synthetic flavor oils and flavoring aromatics and/or natural oils, extracts from plants, leaves, flowers, fruits and so forth and combinations thereof. These may include cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil. Also useful as flavoring agents are vanilla, citrus oil, including lemon, orange, banana, grape, lime and grapefruit, and fruit essences, including apple, pear, peach, strawberry, raspberry, cherry, plum, pineapple, apricot and so forth.

The present invention also contemplates selection of a flavoring agent that is complimentary to the type, kind, amount, etc., of active ingredient(s) used. For example, particularly useful for sulfur containing drugs and, in particular Modafinil, are flavors including mint and fruit punch.

Flavoring agents may be used in conventional amounts, and preferably in an amount ranging from about 0% to about 20% by weight of the dosage form, and more preferably from about 1% to about 10% by weight of the dosage form, and most preferably from about 3% to about 8% by weight of the dosage form.

Coloring agents can be anything known to be used as a coloring agent. Coloring agents useful in the present invention may include titanium dioxide, and dyes suitable for food such as those known as F. D. & C. dyes and natural coloring agents such as grape skin extract, beet red powder, beta-carotene, annatto, carmine, turmeric, paprika, etc. Coloring may be used in conventional amounts, and preferably in an amount ranging from about 0% to about 5% by weight of the dosage form.

Lubricants can be anything known to be used as a lubricant. Lubricants that may be useful in the present invention may include intrinsic or extrinsic lubricants. Intrinsic lubricants may include magnesium, calcium, zinc salts of stearic acid, hydrogenated and partially hydrogenated vegetable oils, animal fats, polyethylene glycol, polyoxyethylene monostearate, talc, light mineral oils, sodium benzoate, sodium lauryl sulphate, magnesium oxide and the like. Lubricants may be used in conventional amounts, and preferably in an amount from about 0% to about 5% by weight of the dosage form.

Disintegrants useful in the present invention may be effervescent or non-effervescent. Effervescent disintegration agents useful in the present invention can be anything known to be used as an effervescent disintegration, such as described in Wehling et al., U.S. Patent No. 5,178,878 cols.5-7, incorporated by reference herein. The acid sources or acid for the effervescent agent may be any which are safe for human consumption and may generally include food acids, acid anhydrides and acid salts. Food acids include citric acid, tartaric acid, malic acid, fumaric acid, adipic acid, and succinic acids etc. Because these acids are directly ingested, their overall solubility in water is less important than it would be if the effervescent tablet formulations of the present invention were intended to be dissolved in a glass of water. Acid anhydrides and acid of the above described acids may also be used. Acid salts may include sodium, dihydrogen phosphate, disodium dihydrogen pyrophosphate, acid citrate salts and sodium acid sulfite.

Carbonate sources include dry solid carbonate and bicarbonate salts such as sodium bicarbonate, sodium carbonate, potassium bicarbonate and potassium carbonate, magnesium carbonate and sodium sesquicarbonate, sodium glycine carbonate, L-lysine carbonate, arginine carbonate and amorphous calcium carbonate.

The effervescent disintegration agent(s) of the present invention is not always based upon a reaction which forms carbon dioxide. Reactants which evolve oxygen or other gasses which are pediatrically safe are also considered within the scope. Where the effervescent agent includes two mutually reactive components, such as an acid source and a carbonate source, it is preferred that both components react completely. Therefore, an equivalent ratio of components which provides for equal equivalents is preferred. For example, if the acid used is diprotic, then either twice the amount of a mono-reactive carbonate base, or an equal amount of a di-reactive base should be used for complete neutralization to be realized. However, in other embodiments of the present invention, the amount of either acid or carbonate source may exceed the amount of the other component. This may be useful to enhance taste and/or performance of a tablet containing an overage of either component. In this case, it is acceptable that the additional amount of either component may remain unreacted.

In general, the amount of effervescent disintegration agent of the present invention useful for the formation of a dosage form according to the present invention should range from about 0% to about 10% of the final composition.

Non-effervescent disintegrants can be anything known to be used as non-effervescent disintegrants. Some non-effervescent disintegrants that may be useful in the present invention include microcrystalline cellulose, (AVICEL PH 200, AVICEL PH 113, AVICEL PH 101), AC-Di-Sol (Croscaramellose Sodium) and PVP-XL (a crosslinked polyvinylpyrrolidone); starches and modified starches; polymers; hydroxyalkyl cellulose such as hydroxymethylcellulose, hydroxypropylcellulose, and hydroxyopropylmethylcellulose, as well as compounds such as carbopol; sweeteners; clays, such as bentonite; alginates; gums such as agar, Arabic, xanthan, guar, locust bean, karaya, pecitin and tragacanth. Non-effervescent disintegrants may be used in conventional amounts and preferably in an amount of about 0% to about 15% by weight of the dosage form.

Viscosity modifiers can be anything known to used as a viscosity modifier. Some viscosity modifiers that may be useful in the present invention include, without limitation, sodium alginate, hydroxypropyl methylcellulose (HPMC), hydroxyethylcellulose (HEC), sodium carboxymethycellulose (sodium CMC), polyvinylpyrrolidone (PVP), Konjac flour, carrageenan, xanthan gum, other hydrophilic polymers, or mixtures thereof. Viscosity modifiers can be used in conventional amounts and preferably in an amount of about 0% to about 5%, and more preferably in an amount of about 0.05% to about 0.5% by weight of the extra granular matrix.

Surfactants can be anything known to be used as surfactants: anionic, cationic, amphoteric, nonionic surfactants such as sucroesters 7-11-15; polysorbates 20-60-80; poloxamers 188-407; sorbitan stearate, etc. Some surfactants that may be useful in the present invention include, without limitation, various grades of the following commercial products: Arlacel®, Tween@, Capmul®, Centrophase®., Cremophor®, Labrafac®, Labrafil®, Labrasol®, Myverol®, Tagat®, and any non-toxic short and medium chain alcohols. Surfactants can be used in conventional amounts and preferably in an amount of about 0% to about 10%, and more preferably in an amount of about 0.5% to about 5% by weight of the extra granular matrix.

Buffers can be anything known to be used as a buffer. Some buffers that may be useful in the present invention include any weak acid or weak base or, preferably, any buffer system that is not harmful to the gastrointestinal mucosa. These include, but are not limited to, any of the acids or bases previously mentioned as the effervescent components, sodium carbonate, potassium carbonate, potassium carbonate, disodium hydrogen phosphate, sodium dihydrogen phosphate, and the equivalent potassium salts. Buffers can be used in conventional amounts and preferably in an amount of about 0.1 % to about 5%, and more preferably in an amount of about 0.2% to about 2% by weight of the matrix.

As long as a resultant dosage form meets the overall objectives of the present invention, and contains a therapeutically effective amount of at least one active ingredient, the amount of matrix is not critical. It is contemplated that the amount of matrix, and the ingredients comprising that matrix will vary.

It has been found that to provide adequate properties using standard techniques and especially with high dose active ingredients, may require an overly high volume of excipient, coating, fillers, etc, rendering the resulting tablet too large. Lyophilization and granulation in accordance with the invention overcomes these problems. Not wishing to be bound by any particular theory, lyophilization as used herein reduces the amount of excipients and residence time in the mouth. This combination, along with the use of a protective granulation binder which additionally affords some level of taste masking, allows for a relatively high amount of active ingredient without leaving a bad taste in the mouth.

Although lyophilization is preferred, it is contemplated that any method may be used to produce dosage forms as long as it results in a dosage form in accordance with the present invention. In one embodiment of the present invention there is provided a method of making an orally dissolvable/disintegrable lyophilized dosage form. While the ingredients and steps may be added or performed in any order, generally, dosage forms can be produced by the steps of granulating an active ingredient and at least one protective granulation binder with or without other ingredients including excipients to produce a protected granulate, mixing the resulting protected granulate with a matrix to form a blend, and lyophilizing said blend into a desired form including tablet, wafer, caplet, etc. Coating of the resulting dosage form, if desired, may be accomplished in any known way.

In another embodiment, the mixture also includes at least one flavoring agent. In another embodiment, the method also includes the step of placing said mixture into a portion of a container prior to lyophilzation and sealing said mixture into said dosage form after lyophilization.

The protected granulate is preferably made by a wet granulation process, but may also be produced by a dry granulation process. Wet granulation is the process in which a liquid is added to a powder in a vessel equipped with any type of agitation that will produce agglomeration or granules of a desired particle size. (Lieberman et al., Pharmaceutical Dosage Forms: Tablets Vol.1, Marcel Dekker, Inc. p. 148-151 (1989). Active ingredients having a high dosage and poor flow and/or compressibility are often wet granulated. *Id.* Dry granulation uses mechanical stress, applied through crushing between two surfaces, direct impact, or cutting to reduce the size of a particulate matter to a desired particle size. Kirk-Othmer Encyclopedia of Chemical Technology, Vol. 22, 4th Ed., John Wiley & Sons, p. 279-296 (1997). No matter which granulation method is used, once the granules are produced, it is contemplated that the granules may be either dried or wetted.

In a preferred embodiment, after milling, the protected granulate has an average particle size of about 1 to about 1000 microns, and more preferably about 10 to about 800 microns, and most preferably about 20 to about 600 microns when tested by a sieve-shaking method. In another preferred embodiment, the protected granulate preferably has a particle size distribution wherein no more than about 10% are less than 45 microns, and no more than 10% are larger than 600 microns. Of course overs and unders could be discarded.

The protected granulate, and in some embodiments, the matrix are combined with a lyophilization solvent prior to lyophilization. Lyophilization solvents that may be useful in accordance with this invention may include but are not limited to water, isopropyl alcohol, ethyl alcohol, tributyl alcohol, or combinations thereof. The solvent may also contain other ingredients such as flavors, sweeteners, polymers, binders, colorants, buffers and surfactants. The protective granulation binder should be selected such that it will not dissolve in the solvent, or should dissolve sufficiently slowly to provide substantial protection during exposure to lyophilization solvents.

Likewise, the active ingredient should be selected such that it will be substantially protected by the protective granulation binder. In one preferred embodiment, the protective granulation binder is selected to be used in conjunction with an active that is substantially insoluble in the selected lyophilization solvent. For example, the granulation binder Eudragit E-100 is preferred to be used with Modafinil, famotidine or fentanyl and/or salts thereof, and other such drugs which are insoluble in water. In one aspect of the present invention it is contemplated that some amount of solvent may be detected in the resulting dosage form, however it is preferred that the dosage form contain little if any residual solvent.

The blend of matrix materials, protected granulate and lyophilization solvent may be mixed for any period of time, under any conditions, using any kind of conventional blending apparatus to form a blend in accordance with this invention. Generally blending is done under conditions that would not alter the desirable properties of the coated particles or matrix materials, such as, without limitation, radically altering the particle size distribution. While it is generally desirable that the mixture be homogeneous, particularly where content uniformity is an issue, there might be instances where a random or patterned distribution is contemplated. Thus, blending could be more broadly thought of as any mixing operation.

The blend may then be poured into molds or shaped into a variety of forms for administration to a patient and lyophilized. Lyophilization is performed as described Lafon, U.S. Patent No. 4,616,047, Nguyen et al., U.S. Patent No. 5,843,347, and Blonde et al., U.S. Patent No. 3,855,712, all of which are herein incorporated by reference. Generally speaking, all of the raw materials, including the protected granulate, are weighed and then a solution is prepared of the various soluble ingredients. A suspension of the protected particles and other insoluble materials, possibly with homogenization, is accomplished using a mixer or stirrer and then the suspension is poured into preformed blisters. These are then loaded into a lyophilizer wherein the materials are first frozen and then freeze dried.

Once lyophilized, it is contemplated that the dosage forms may be stamped, painted, coated, printed, etc. These dosage forms may be stored in bulk, in blister packs, in conventional openable and reclosable multi-tablet bottles or other similar packaging.

The present invention is a delivery vehicle contemplated to be used to administer any active ingredient, and particularly those where high dosages (> 50mg) of bad tasting compounds are required. It is especially useful for persons who have difficulty swallowing, geriatric persons, children, persons with disabilities, etc. The dosage form may be placed in the mouth, allowing the patient's saliva to disintegrate/dissolve the dosage form into an organoleptically pleasant slurry/solution/suspension to be easily swallowed.

The frequency of dosing depends on various factors including the amount of active ingredient present in the dosage form, the size of the dosage form, the weight of the patient, the condition of the patient, side effects of the active ingredient, etc. The administration of multiple dosage forms and multiple frequency of dosing is contemplated depending upon the above factors as well as duration of the patient's condition, how long the active ingredient stays in a patient's system, etc. It is also contemplated that at least one dosage form in accordance with the present invention will be administered to a patient at least once in month time period. Such administration would include on factors given above as well as the patient's condition, the age and size of the patient, the amount of time the active ingredient stays in a patient's system, the type and kind of side effects, etc.

### EXAMPLES

Example 1

| Materials | % |
|---|---|
| Modafinil | 92 |
| Eudragit E | 8 |

Granules were manufactured in a high shear granulator where Modafinil powder was loaded into the granulator bowl and Eudragit E alcoholic solution (30%) was added slowly. The impeller and chopper speeds were maintained at pre-selected values to provide enough shear for granule formation and growth. The granules were then wet milled and loaded into the fluid bed to be dried to <1% LOD. Of the resultant granules, 70% by weight were between 750 microns and 250 microns determined by a sieve-shaking method.

Example 2

**Strength: 425 mg Modafinil (lyophilized ODT)**

| Ingredient | % | Unit formula | Batch formula 5000 units |
|---|---|---|---|
| Modafinil granules (potency 92%) | 77.00% | 462.0 mg | 2310.0 g |
| Sucralose | 3.33% | 20.0 mg | 100.0 g |
| Dextran 70 | 4.16% | 25.0 mg | 125.0 g |
| Xanthan gum | 0.25% | 1.5 mg | 7.5 g |
| Grape flavor | 5.00% | 30.0 mg | 150.0 g |
| Strawberry flavor | 3.33% | 20.00 mg | 100.0 g |
| Disodium hydrogen phosphate | 0.50% | 3.0 mg | 15.0 g |
| Lactose monohydrate | 6.42% | 38.5 mg | 192.5 g |
| Purified water | | 700.0 mg | 3500.0 g |
| Total wet weight | | 1300.0 mg | 6500.0 g |
| Total unit weight | | 600.0 mg | |

Xanthan gum, disodium hydrogen phosphate (Na2HPO4 anhydrous) were added to water and stirred at RT until a homogeneous solution was obtained.

Modafinil granules having 92% potency, as prepared in Example 1, sucralose, dextran 70, lactose, flavor (grape and strawberry) were then introduced into a mixer equipped with a vacuum system. The blend powder was mixed at atmospheric pressure (~ 1000 HPa) up to about 15 minutes and under low pressure (200-400 HPa) up to about 15 minutes.

The Xanthan gum/Na2HPO4 solution was then introduced in the mixer at low pressure (~ 200 HPa) for a few minutes.

The wet suspension was mixed for approximately 60 minutes at atmospheric pressure and RT, and then for a few minutes at low pressure (200-250HPa) and then again for at least 30 minutes at atmospheric pressure (~ 1000 HPa).

The homogenous suspension was distributed into preformed PVC blister. Unit weight and blister weight were monitored during the filling of blisters.

The blisters filled with suspension were then introduced into the freeze-dryer and frozen at a temperature below - 25°C for at least 30 minutes, then freeze-dried for approximately 240 minutes. This process was followed by secondary drying for at least 90 minutes at a maximum temperature of approximately +60°C.

The blisters filled with lyophilized product were then sealed with an aluminum foil in a controlled room (RH< 40%) (T°= 18°-22°C). Appearance, hardness, friability, disintegration time, water content and weight uniformity were monitored for the lyophilized product.

The resulting tablets met the following specifications:

Appearance: White to off white solid oral dosage form easy to extract from blister.

Hardness >15 N

Friability <5% after 50 rotations

Disintegration time (n=6) Average < 60 seconds

Water content <2%

Weight uniformity: Theoretical mass ± 5%

Example 3

The same process as described in Example 2 and the granules of Example 1 above were used herein to produce dosage forms having the following composition.

**Strength: 425 mg Modafinil lyoc (lyophilized ODT)**

| Ingredient | % | Unit formula | Batch formula 5000 units |
|---|---|---|---|
| Modafinil granules (potency 92 %) | 77.00% | 462.0 mg | 2310.0 g |
| Sucralose | 3.33% | 20.0 mg | 100.0 g |
| Dextran 70 | 4.16% | 25.0 mg | 125.0 g |
| Xanthan gum | 0.25% | 1.5 mg | 7.5 g |
| Orange flavour | 8.33% | 50.0 mg | 250.0 g |
| Disodium hydrogen phosphate | 0.33% | 2.0 mg | 10.0 g |
| Lactose monohydrate | 6.33% | 39.5 mg | 197.5 g |
| Purified water | | 700.0 mg | 3500.0 g |
| Total wet weight | | 1300.0 mg | 6500.0 g |
| Total unit weight | | 600.0 mg | |

Example 4

The same process as described in Examples 2 and 3 above, was used to make dosage forms for the following formulations.

**Compositions Selected from Lab Scale Development**

| Strength 425 mg | Grape | | Mixed Berry | | Orange | |
|---|---|---|---|---|---|---|
| Formulation Number | 1 | | 2 | | 3 | |
| | mg | % | mg | % | mg | % |
| Modafinil granules (potency ~92%) | 462 | 77.00% | 462 | 77.00% | 462 | 77.00% |
| Sucralose | 20 | 3.33% | 20 | 3.33% | 20 | 3.33% |
| Dextran 70 | 20 | 3.33% | 20 | 3.33% | 20 | 3.33% |
| Xanthan gum | 1.5 | 0.25% | 1.5 | 0.25% | 1.5 | 0.25% |
| Grape flavor | 50 | 8.33% | 30 | 5.00% | - | - |
| Strawberry flavor | - | - | 20 | 3.33% | - | - |
| Orange flavor | - | - | - | - | 50 | 8.33% |
| Disodium hydrogenophosphate (Na2HPO4) | 5 | 0.83% | 3 | 0.50% | 2 | 0.33% |
| Lactose | 41.5 | 6.92% | 43.5 | 7.25% | 44.5 | 7.42% |
| Purified water | 700 | - | 700 | - | 700 | - |
| | | | | | | |
| Unit suspension weight | 1300 | - | 1300 | - | 1300 | - |
| Unit weight | 600 | - | 600 | - | 600 | - |

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. An orally dissolvable/disintegrable lyophilized dosage form comprising:
a protected granulate comprising at least one first active ingredient and at least one protective granulation binder; and a matrix, said dosage form being a lyophilized, orally dissolvable/disintegrable dosage form adapted for direct oral dosing in the mouth of a patient.

2. The dosage form of claim 1, wherein said active ingredient is a sulfur containing compound.

3. The dosage form of claim 1, wherein said active ingredient is modafinil, famotidine or fentanyl and/or salts thereof.

4. The dosage form of claim 1, wherein said protective granulation binder will dissolve at basic pH.

5. The dosage form of claim 1, wherein said protective granulation binder will dissolve at neutral pH.

6. The dosage form of claim 1, wherein said protective granulation binder will dissolve at acidic pH.

7. The dosage form of claim 1, wherein said protective granulation binder will dissolve at a pH of about 7 or less.

8. The dosage form of claim 1, wherein said matrix comprises at least one of a binder, lyophilization binder, filler, sugar, artificial sweetener, polymer, flavoring agent, taste masking material, active ingredient, coloring agent, lubricant, effervescent disintegrant, non-effervescent disintegrant, viscosity modifier, surfactant, and buffer.

9. The dosage form of claim 1, further comprising at least one flavor selected to be complimentary with the active ingredient such as to provide a pleasant organoleptic sensation to the mouth.

10. The dosage form of claim 9, wherein said flavor is selected from the group consisting of fruit punch, orange, banana, cherry, mint, bubble gum, grape, paradise punch, peach, mango, and combinations thereof.

11. The dosage form of claim 1, wherein said active ingredient is present in an amount of about 0.01 mg to about 2g by weight of the dosage form.

12. The dosage form of claim 1, wherein said active ingredient is present in an amount of about 0.05mg to about 1 g by weight of the dosage form.

13. The dosage form of claim 1, wherein said active ingredient is present in an amount of about 1 mg to about 800mg by weight of the dosage form.

14. The dosage form of claim 1, wherein said active ingredient is present in an amount of about 85mg to about 425mg.

15. The dosage form of claim 1, wherein said active ingredient is present in an amount of about 50mg or greater.

16. The dosage form of claim 1, wherein said protected granulate is provided in an amount of about 0.1 % to about 90% by weight of the dosage form.

17. The dosage form of claim 1, wherein said protected granulate is provided in an amount of about 1 % to about 80% by weight of the dosage form.

18. The dosage form of claim 1, wherein said protected granulate further comprises at least one excipient.

19. The dosage form of claim 18, wherein said excipient is a binder, pH adjusting substance, filler, disintegrant, solid support or buffer.

20. The dosage form of claim 1, wherein said protective granulation binder is provided in an amount of about 0.1 % to about 50%, based on the weight gain of said protected granulate.

21. The dosage form of claim 1, wherein said protective granulation binder is provided in an amount of about 2% to about 25%, based on the weight gain of said granulate.

22. The dosage form of claim 1, wherein said protective granulation binder is provided in an amount of about 5% to about 15% based on the weight gain of said granulate.

23. The dosage form of claim 1, wherein said protective granulate has an average particle size of about 1 to about 1000 microns.

24. The dosage form of claim 1, wherein said protective granulate has an average particle size of about 10 to about 800 microns.

25. The dosage form of claim 1, wherein said protective granulate has an average particle size of about 20 to about 600 microns.

26. The dosage form of claim 1, wherein said protective granulate has a particle size distribution wherein no more than about 10% are less than 45 microns and no more than about 10% are larger than 600 microns.

27. The dosage form of claim 1, wherein said protective granulate has a particle size distribution wherein no more than about 10% are less than 75 microns and no more than about 10% are larger than 425 microns.

28. The dosage form of claim 1, wherein said active ingredient is generally water insoluble.

29. A method of making an orally dissolvable/disintegrable lyophilized dosage for direct oral dosing form comprising the steps of:
granulating at least one active ingredient and at least one protective granulation binder to produce a protected granulate;
mixing said protected granulate with a matrix and a lyophilization solvent to form a blend; and
lyophilizing said blend to form a dosage form.

30. The method of claim 29, further comprising the step of placing said blend into a portion of a container prior to lyophilization and sealing said dosage form into said container after lyophilization.

31. The method of claim 29, further comprising the step of adding an organoleptically complimentary pleasant flavor during mixing said protected granulate with a matrix to form a mixture.

32. The method of claim 31, wherein said flavor is selected from the group consisting of fruit punch, orange, banana, cherry, mint, bubble gum, grape, paradise punch, peach, mango, and combinations thereof.

33. A method of treating a patient comprising the steps of:
placing an orally dissolvable/disintegrable lyophilized dosage form of claim 1 into the mouth of a patient in need of treatment;
allowing said dosage form to disintegrate/dissolve sufficiently in the mouth of a patient to form a solution, suspension or slurry; and
swallowing said solution suspension or slurry.
